# Europäisches Patentamt
# European Patent Office
# Office européen des brevets

(19)

(11) Veröffentlichungsnummer: **0 280 055 B1**

(12) **EUROPÄISCHE PATENTSCHRIFT**

(45) Veröffentlichungstag der Patentschrift:
26.09.90

(51) Int. Cl.⁵: **C07C 15/24**, C07C 2/86, B01J 29/28

(21) Anmeldenummer: **88101145.6**

(22) Anmeldetag: **27.01.88**

(54) Verfahren zur Herstellung von 2,6-Dialkylnaphthalin.

(30) Priorität: **04.02.87 DE 3703291**

(43) Veröffentlichungstag der Anmeldung:
**31.08.88 Patentblatt 88/35**

(45) Bekanntmachung des Hinweises auf die Patenterteilung:
**26.09.90 Patentblatt 90/39**

(84) Benannte Vertragsstaaten:
**BE DE FR GB NL**

(56) Entgegenhaltungen:
**DE-A- 3 334 084**

**CHEMICAL ABSTRACTS, Band 104, Nr. 7, 17. Februar 1986, Seite 501, Zusammenfassung Nr. 50695s, Columbus, Ohio, US; & JP-A-60 172 937**

(73) Patentinhaber: **RÜTGERSWERKE AKTIENGESELLSCHAFT, Mainzer Landstrasse 217, D-6000 Frankfurt am Main 1(DE)**

(72) Erfinder: **Weltkamp, Jens, Prof. Dr., Rotkehlchenweg 4, D-2900 Oldenburg(DE)**
Erfinder: **Neuber, Marita, Rudolfstrasse 1, D-7500 Karlsruhe 1(DE)**
Erfinder: **Höltmann, Wilhelm, Dr., Dorffeldstrasse 9, D-4400 Münster(DE)**
Erfinder: **Collin, Gerd, Dr., Hagensallee 56, D-4100 Duisburg(DE)**
Erfinder: **Spengler, Hans, Dr., Windmühlenberg 3, D-4716 Olfen(DE)**

## Beschreibung

Die Erfindung betrifft ein Verfahren zur Herstellung von 2,6-Dialkylnaphthalin durch selektive Alkylierung von Naphthalin oder 2-Alkylnaphthalin. 2,6-Dialkylnaphthaline sind u. a. wertvolle Ausgangsprodukte für Naphthalin-2,6-dicarbonsäure, die zur Herstellung von hochwertigen Polyester- oder Polyamidtypen dient.

Aus DE-A 33 34 084 ist bekannt, daß es gelingt, Naphthalin oder Alkylnaphthaline mit Methanol oder Dimethylether an einem Zeolith-Katalysator vom Pentasil-Typ, bevorzugt ZSM-5, zu methylieren. Die Umsetzung erfolgt bei Temperaturen zwischen 350 und 600 °C, bevorzugt zwischen 400 und 550 °C.

Gemäß der in den Beispielen gegebenen Werte führt die Umsetzung von Naphthalin mit Methanol in guter Ausbeute und hoher Selektivität zu 2-Methylnaphthalin.

Die Umsetzung von 2-Methylnaphthalin mit Methanol ergibt, ebenfalls in hoher Ausbeute und Selektivität, als Hauptprodukt 2,6-Dimethylnaphthalin - neben allerdings beachtlichen Mengen an 1-Methylnaphthalin.

Beim Nacharbeiten des Verfahrens zeigten sich Nachteile. Insbesondere ergab sich, daß die Mengenangaben für 2,6-Dimethylnaphthalin auch das 2,7-Dimethylnaphthalin beinhalten, das mittels herkömmlicher gaschromatographischer Methoden von 2,6-Dimethylnaphthalin kaum zu trennen ist. Beide Isomere sind aber in etwa gleichen Anteilen im Reaktionsprodukt enthalten.

Auch nach einem aus C.A. 104: 50695a bekannten Verfahren zur Alkylierung von Naphthalin an einem Gemisch aus einem $H^+$-Zeolithen und gamma-Aluminiumoxid entstehen 2,6- und 2,7-Dimethylnaphthalin im Verhältnis 41,2 zu 37,8 %.

Es ist daher Aufgabe der Erfindung, ein wirtschaftliches Verfahren zur Herstellung von 2,6-Dialkylnaphthalin bereitzustellen, bei dem eine lange Standzeit des Katalysators gegeben ist, und bei dem das 2,6-Dialkylnaphthalin in möglichst hoher Ausbeute und Selektivität gebildet wird.

Die Lösung der Aufgabe erfolgt durch ein Verfahren gemäß der Ansprüche 1 bis 11.

Es wurde gefunden, daß bei der Durchführung der Alkylierungsreaktion im Temperaturbereich, der um 5 bis 100 °C niedriger ist als es dem Stand der Technik entspricht, die Alkylierungs-Aktivität des Katalysators nur geringfügig sinkt, so daß eine lange Standzeit des Katalysators bei nahezu gleichbleibendem Umsatz gegeben ist.

Überraschenderweise wurden darüber hinaus weitere Effekte gefunden, die wesentlich zur besseren Wirtschaftlichkeit des erfindungsgemäßen Verfahrens beitragen:

Mit abnehmender Reaktionstemperatur verschieben sich die Ausbeuten zugunsten von 2,6-Dialkylnaphthalin. Die Reaktion wird selektiver. So liegt z. B. das Verhältnis von 2,6-Dimethylnaphthalin zu den Dimethylnaphthalinen insgesamt nach 2 Stunden Versuchsdauer bei 400 °C bei 0,42 und nach 2 Stunden Versuchsdauer bei 300 °C bei 0,52.

Bei Einsatz von 2-Alkylnaphthalin nimmt mit abnehmender Reaktionstemperatur die Bildungsgeschwindigkeit von 1-Alkylnaphthalin stärker ab als die der Alkylierungsprodukte. Die Selektivität für das unerwünschte Nebenprodukt 1-Alkylnaphthalin wird also geringer.

Die störende Nebenreaktion der Isomerisierung von 2-Alkylnaphthalin, d.h. die Bildung von 1-Alkylnaphthalin, tritt bevorzugt am frischen Katalysator auf.

Im Verlauf der Reaktion werden offensichtlich die Katalysatorzentren, die die störende Isomerisierung katalysieren, blockiert. Der Umsatz an 2-Alkylnaphthalin entspricht dann nahezu der Bildung von Dimethylnaphthalin. Der Anteil an z. B. 1-Methylnaphthalin im Reaktionsgemisch, der zu Beginn der Umsetzung bei etwa 4,5 % liegt, sinkt relativ schnell ab und beträgt nach 12 Stunden (340 °C) nur noch weniger als 1 %.

Es wurde gefunden, daß diese Änderung der Transalkylierungsaktivität durch die Bildung von Kohlenstoff-Rückstand auf der Katalysatoroberfläche, d. h., durch Verkokung von Teilen der eingesetzten Reaktionspartner am Katalysator bedingt ist.

Es wurde weiterhin gefunden, daß diese unerwünschte Isomerisierung weitgehend vermieden werden kann, und die Bildung von 1-Alkylnaphthalin unter 0,1 % gesenkt werden kann, wenn bei der Alkylierungsreaktion als Katalysator bereits ein vorverkokter Zeolith eingesetzt wird. Dazu wird vor der Alkylierung von 2-Alkylnaphthalin eine organische Verbindung am selben Zeolith umgesetzt. Um die Entstehung eventuell die geplante Umsetzung störender oder verunreinigender Komponenten zu vermeiden, bieten sich als Reaktanden für die Verkokung Alkohole, Olefine oder Alkylnaphthaline an, insbesondere die, die bei der folgenden Umsetzung als Reaktanden dienen.

Diese Vorverkokung des Katalysators kann im Temperaturbereich von 250 bis 600 °C erfolgen, bevorzugt zwischen 300 bis 450 °C. Damit sich eine ausreichende Menge Koks auf dem Katalysator und/oder in seinen Poren bilden kann, um die Isomerisierung von 2-Alkylnaphthalin zurückzudrängen, soll pro Gramm trockenen Katalysators eine solche Menge an Reaktand umgesetzt werden, daß zwischen 1 und 8 Gramm Kohlenstoff aufgegeben werden. Die Verkokung der Katalysatoren wird geeigneterweise in demselben Reaktor durchgeführt, in dem im Anschluß die Alkylierung durchgeführt wird. Die Reaktanden können dabei entweder als Flüssigkeit oder in einem Teil als Reaktors verdampft und gasförmig, gegebenenfalls mit Hilfe eines inerten Trägergases, zudosiert werden. Direkt nach der Verkokung kann mit der Alkylierung von 2-Alkylnaphthalin begonnen werden.

Der Umsatz ist abhängig von der Kristallitgröße des eingesetzten Zeoliths. So ergeben z. B. vergleichbare Zeolithe mit unterschiedlicher Kristallitgröße unter gleichen Bedingungen bei der Alkylierung von 2-Methylnaphthalin mit Methanol folgende Umsätze an 2-Methylnaphthalin:

Kristallitgröße und Umsatz von 2-Methylnaphthalin (%) an HZSM-5 bei 340 °C:

| Reaktionszeit (h) | 0,25 | 1 | 2 | 5 | 10 |
|---|---|---|---|---|---|
| 1 µm | 20 | 17,5 | 15,5 | 13 | 11 |
| 2 µm | 11,5 | 9,5 | 8,3 | 7 | 6 |
| 16 µm | 6,5 | 5,5 | 4,7 | 4 | 3,5 |

Die Kristallitgröße, d. h. der durchschnittliche Durchmesser der Zeolithkristallite kann im Bereich von 0,01 bis 20 µm liegen. Bevorzugt wird ein Bereich von 0,05 bis 2 µm, der einerseits mit vertretbarem Aufwand erreicht wird, andererseits aber auch bei niedrigen Temperaturen gute Umsätze ergibt.

Die Durchführung der erfindungsgemäßen Umsetzung erfolgt in einem an sich bekannten Reaktor, der mit dem Zeolith-Katalysator beschickt ist und der mittels einer Heizung auf die entsprechende Reaktionstemperatur aufgeheizt ist. Die Reaktanden werden entweder als Flüssigkeiten zudosiert und in einem Teil des Reaktors verdampft oder gasförmig, gegebenfalls mit einem inerten Trägergas eingespeist und über den Katalysator geleitet. Die Kontaktzeit beträgt dabei 0,1 bis 0,5 s.

Reaktanden sind Naphthalin oder 2-Alkylnaphthaline wie 2-Methyl-, 2-Ethyl-, 2-Propyl- oder 2-Isopropylnaphthalin einerseits und Alkylierungsmittel wie niedrige Alkohole, Dialkylether oder Alkylhalogenide andererseits. Beispiele für derartige Alkylierungsmittel sind Methanol, Dimethylether, Methylbromid, Ethanol, Ethylchlorid, Propanol oder Isopropanol.

Bevorzugte Reaktanden sind 2-Methylnaphthalin und Methanol oder Dimethylether. 2-Methylnaphthalin. kann entweder rein oder als Gemisch aus 1- und 2-Methylnaphthalin eingesetzt werden. Ferner können Gemische eingesetzt werden, wie sie aus einer vorgeschalteten Methylierung von Naphthalin resultieren und neben 2-Methylnaphthalin als Hauptkomponente auch 1-Methylnaphthalin und bereits Dimethylnaphthaline enthalten. Beim Einsatz von Methylnaphthalingemischen wird als zusätzlicher Vorteil des Verfahrens eine Umalkylierung in dem Sinne beobachtet als mehr 2,6-Dimethylnaphthalin gebildet wird, als es dem Umsatz an 2-Methylnaphthalin entspricht.

Das molare Verhältnis von Methylnaphthalin zu Methanol oder Dimethylether kann in dem weiten Bereich von 10 : 1 bis 1 : 5 variieren. Es ist einleuchtend, daß die Menge an gebildeten Dimethylnaphthalinen bei höherem Anteil an Methanol oder Dimethylether steigt. Andererseits entstehen bei hohem Anteil an Methanol oder Dimethylether verstärkt höher methylierte Naphthaline. Somit liegt das bevorzugte Verhältnis im Bereich von 1 : 1 bis 1 : 0,5.

Katalysatoren für die Alkylierungsreaktion sind Zeolithe verschiedenster Art, insbesondere solche, deren Poren aus 10 oder 12 Sauerstoffatomen gebildet werden, sogenannte 10-Ring- oder 12-Ring-Zeolithe. Es wurde gefunden, daß die effektive Porenweite des Zeoliths entscheidend ist für die selektive Alkylierung von Naphthalin und 2-Alkylnaphthalinen zu 2,6-Dialkylnaphthalinen.

Als Maß für die effektive Porenweite von 12- und 10-Ring-Zeolithen wird zweckmäßigerweise der "Spaciousness Index" (SI) verwendet (vergl. J. Weitkamp, S. Ernst, R. Kumar, Appl. Catal. 27, 207-210 (1986)). Er kann in einfacher Weise mittels einer katalytischen Testreaktion ermittelt werden, indem ein Naphthen mit 10 Kohlenstoffatomen, wie insbesondere Butylcyclohexan oder Pentylcyclopentan, an einer bifunktionellen Form (sauer und hydrier-/dehydrieraktiv) des Zeoliths umgesetzt wird. Der Spaciousness Index ist dann definiert als Quotient der Selektivitäten für Isobutan und n-Butan (SI = $n_{i\text{-Butan}}/n_{n\text{-Butan}}$).

In der Tabelle 1 sind die Werte des Spaciousness Indexes für einige Zeolithe aufgeführt.

| Zeolith | SI |
|---|---|
| Y | 21 |
| ZSM-20 | 21 |
| Beta | 19 |
| L | 17 |
| Mordenit | 7 |
| EU-1 | 5 |
| Offretit | 5 |
| ZSM-12 | 3 |
| ZSM-5 | 1 |
| ZSM-22 | 1 |

Der Spaciousness Index ist ein quantitatives Maß für den Raum, der in einem Zeolith für katalytische Umsetzungen zur Verfügung steht. Je kleiner der SI-Wert ist, desto enger sind die Poren und/oder Hohlräume.

Es wurde gefunden, daß für die erfindungsgemäßen Umsetzungen Zeolithe mit einem Spaciousness Index von 0,6 bis ca. 12 verwendet werden können. Für Methylierungen oder Ethylierungen eignen sich insbesondere Zeolithe mit einem Spaciousness Index von 0,6 bis 5. Dabei sind für die Methylierung von Naphthalin oder 2-Methylnaphthalin besonders Zeolithe vom Pentasiltyp wie vor allem ZSM-5 geeignet.

Für Alkylierungen mit sperrigeren Alkylgruppen, wie etwa der Isopropylgruppe, sind Zeolithe mit einem Spaciousness Index von 2 bis 12 bevorzugt.

Die Zeolithe müssen nach der Synthese in eine katalytisch aktive Form überführt werden. Dazu werden sie bei Temperaturen von 300 bis 700 °C, vorzugsweise im Bereich von 400 bis 600 °C, calciniert, um das organische Templat zu entfernen. Um die aktiven Zentren zu erzeugen, werden durch Ionenaustausch $H^+$ oder $NH_4^+$, Erdalkalionen, wie insbesondere $Mg^{2+}$ oder $Ca^{2+}$, dreiwertige Metallkationen wie z. B. $Ce^{3+}$, $La^{3+}$ oder Kationen der Lanthaniden oder andere mehrwertige Metallkationen eingeführt. Durch Trocknen der ausgetauschten Zeolithe bei 250 bis 600 °C, speziell bei 300 bis 400 °C, werden Säurezentren erzeugt.

Das Si/Al-Atomverhältnis der Zeolithe kann in einem weiten Bereich von ca. 3 bis 1000 variieren, bevorzugt sind Si/Al-Verhältnisse zwischen 15 und 200.

Beispiel:

Beschreibung der Versuchsapparatur:

Die Versuche werden bei Atmosphärendruck in einer Festbett-Strömungsapparatur durchgeführt. Die Reaktanden werden durch temperaturgeregelte Sättiger, die von Stickstoff als Trägergas durchströmt werden, zudosiert. In einem Mischgefäß werden beide Stickstoffströme vereinigt und dann in den Reaktor geleitet. Zum Einstellen der Strömungen kann der Reaktor im Bypass umgangen werden. Der Reaktor ist aus Quarzglas. Die auf einer Fritte liegende Katalysatorschüttung wird von oben durchströmt. Der Reaktor wird durch einen elektrischen Ofen beheizt. Die Probennahme erfolgt on-line. Gleichzeitig werden die Reaktionsprodukte in einer Kühlfalle bei 0 °C auskondensiert. Die Reaktionsprodukte werden durch Kapillargaschromatographie analysiert.

Durchführung der Versuche:

Die Katalysatoren wurden gepreßt, gemahlen und gesiebt. Die Kornfraktion von 0,2 bis 0,3 mm wurde eingesetzt. Die trockene Katalysatormasse betrug 0,19 g. Die Zeolithe wurden in situ im Stickstoffstrom ($V_{N2}$ = 4,5 l/h) bei 500 °C 6 h lang getrocknet.

| Versuchsbedingungen: | |
| --- | --- |
| Katalysator | $NH_4ZSM$-5 |
| Reaktanden | 2-Methylnaphthalin (= 2-MN), $CH_3OH$ |
| Reaktionstemperatur | 300 °C |
| Partialdruck von 2-MN | $p_{2-MN}$ = 0,019 bar |
| Molverhältnis | $n_{2-MN} : n_{CH3OH}$ = 1:0,5 |
| modifizierte Verweilzeit | $W/F_{2-MN}$ = 160 gh/mol |
| Katalysatormasse, trocken | W = 0,19 g |
| Verweilzeit | = 0,2 s |

4

| Ergebnisse: | | | |
|---|---|---|---|
| Versuchsdauer | 0,5 h | 2 h | 8 h |
| Umsatz (%) | 7 | 5 | 5 |
| Ausbeuten (%) | | | |
| Naphthalin | 0,1 | 0,1 | 0,1 |
| 1-Methylnaphthalin | 1,5 | 0,5 | 0,5 |
| Dimethylnaphtaline | 5 | 4 | 4 |
| höher methylierte Naphthaline | 0,5 | 0,5 | 0,5 |
| Zusammensetzung der Dimethylnaphthalin-Fraktion (%) | | | |
| 2,6-Dimethylnaphthalin | 50 | 52 | 55 |
| 2,7-Dimethylnaphthalin | 34 | 33 | 32 |
| 1,3- + 1,7-Dimethylnaphthalin | 4 | 3 | 2 |
| 1,6-Dimethylnaphthalin | 4 | 3 | 2 |
| 1,4- + 2,3-Dimethylnaphthalin | 6 | 7 | 7 |
| 1,5-Dimethylnaphthalin | 0,01 | 0,01 | 0,01 |
| 1,2-Dimethylnaphthalin | 2 | 2 | 2 |

## Patentansprüche

1. Verfahren zur Herstellung von 2,6-Dialkylnaphthalin durch Alkylierung von Naphthalin oder 2-Alkylnaphthalin an einem Zeolith-Katalysator, dadurch gekennzeichnet, daß die Alkylierung bei einer Temperatur im Bereich von 250 bis 345 °C durchgeführt wird.

2. Verfahren nach Anspruch 1, dadurch gekennzeichnet, daß die Alkylierung im Bereich von 300 bis 320 °C durchgeführt wird.

3. Verfahren nach den Ansprüchen 1 bis 2, dadurch gekennzeichnet, daß Naphthalin oder 2-Methylnaphthalin methyliert werden.

4. Verfahren nach den Ansprüchen 1 bis 3, dadurch gekennzeichnet, daß als Zeolithe solche mit einem Spaciousness Index von 1 bis 12 eingesetzt werden.

5. Verfahren nach den Ansprüchen 3 bis 4, dadurch gekennzeichnet, daß als Katalysatoren ZSM-5-Zeolithe eingesetzt werden.

6. Verfahren nach den Ansprüchen 1 bis 5, dadurch gekennzeichnet, daß die Kristallitgröße des Zeoliths im Bereich von 0,01 bis 20 μm liegt.

7. Verfahren nach Anspruch 6, dadurch gekennzeichnet, daß die Kristallitgröße des Zeoliths im Bereich von 0,05 bis 5 μm liegt.

8. Verfahren nach den Ansprüchen 1 bis 7, **dadurch gekennzeichnet**, daß als Katalysator ein vorverkokter Zeolith eingesetzt wird.

9. Verfahren nach den Ansprüchen 1 bis 8, **dadurch gekennzeichnet**, daß als Katalysator ein Zeolith eingesetzt wird, der durch Behandlung mit einem Alkohol, einem Olefin oder einem Alkylnaphthalin bei Temperaturen im Bereich von 250 bis 600 °C vorverkokt ist.

10. Verfahren nach den Ansprüchen 8 und 9, **dadurch gekennzeichnet**, daß der Zeolith so vorverkokt wird, daß pro Gramm trockenen Katalysators eine solche Menge an Reaktand umgesetzt wird, daß 1 bis 8 Gramm Kohlenstoff aufgegeben werden.

## Claims

1. A process for the preparation of 2,6-dialkyl naphthalene by alkylation of naphthalene or 2-alkyl naphthalene on a zeolite catalyst, characterized in that the alkylation is carried out at a temperature in the range of from 250 to 345°C.

2. A process according to Claim 1, characterized in that the alkylation is carried out in the range of from 300 to 320°C.

3. A process according to Claims 1 and 2, characterized in that naphthalene or 2-methyl naphthalene is methylated.

4. A process according to Claims 1 to 3, characterized in that zeolites with a spaciousness index of from 1 to 12 are used.

5. A process according to Claims 3 and 4, characterized in that ZSM-5-zeolites are used as catalysts.

6. A process according to Claims 1 to 5, characterized in that the crystallite size of the zeolite is in the range of from 0.01 to 20 µm.

7. A process according to Claim 6, characterized in that the crystallite size of the zeolite is in the range of from 0.05 to 5 µm.

8. A process according to Claims 1 to 7, characterized in that a pre-coked zeolite is used as the catalyst.

9. A process according to Claims 1 to 8, characterized in that a zeolite, which is pre-coked by treatment with an alcohol, an olefin or an alkyl naphthalene at temperatures in the range of from 250 to 600°C, is used as the catalyst.

10. A process according to Claims 8 and 9, characterized in that the zeolite is pre-coked in such a way that such a quantity of reactand is reacted per gramme of dry catalyst that from 1 to 8 grammes of carbon are delivered.

**Revendications**

1. Procédé pour la préparation de 2, 6-dialkylnaphtalène par alkylation de naphtalène ou de 2-alkylnaphtalène sur un catalyseur à la zéolithe, caractérisé en ce que l'alkylation est conduite à une température dans la gamme de 250 à 345°C.

2. Procédé selon la revendication 1, caractérisé en ce que l'alkylation s'effectue dans la gamme de 300 à 320°C.

3. Procédé selon les revendications 1 à 2, caractérisé en ce qu'on méthyle du naphtalène ou du 2-méthylnaphtalène.

4. Procédé selon les revendications 1 à 3, caractérisé en ce qu'on met en œuvre comme zéolithes celles possédant un spaciousness index de 1 à 12.

5. Procédé selon les revendications 1 à 4, caractérisé en ce qu'on met en œuvre comme catalyseurs des zéolithes ZSM-5.

6. Procédé selon les revendications 1 à 5, caractérisé en ce que la dimension de cristallite de la zéolithe se situe dans la gamme de 0, 01 à 20 µm.

7. Procédé selon la revendication 6, caractérisé en ce que la dimension de cristallite de la zéolithe se situe dans la gamme de 0, 05 à 5 µm.

8. Procédé selon les revendications 1 à 7, caractérisé en ce qu'on met en œuvre comme catalyseur une zéolithe précokéfiée.

9. Procédé selon les revendications 1 à 8, caractérisé en ce qu'on met en œuvre comme catalyseur une zéolithe qui est précokéfiée par traitement avec un alcool, une oléfine ou un alkylnaphtalène à des températures dans la gamme de 250 à 600°C.

10. Procédé selon les revendications 8 et 9, caractérisé en ce que la zéolithe est précokéfiée de manière que, par gramme de catalyseur sec, il réagisse une quantité de réactif telle qu'il se dépose 1 à 8 grammes de carbone.